# EUROPEAN PATENT APPLICATION

(11) **EP 1 391 179 A1**
(43) Date of publication of application: **25.02.2004**
(21) Application number: 02447148.4
(22) Date of filing: 30.07.2002
(51) Int. Cl.: A61B 5/22, A61B 5/00, A63B 24/00

(54) **Exercise manager program**

(71) Applicant: Kostucki, Willy, 1190 Bruxelles (BE)
(72) Inventor: Kostucki, Willy, 1190 Bruxelles (BE)

(57) **Abstract**

The innovation consists of a bidirectional link between the specialist in cardiovascular disease and the cardiovascular training device used by the patient. This *monitoring loop* is materialized either by a *memory card or an Internet connection*, which connects two programs, one on the doctor's computer desktop and the second into the training device itself. There a *minimum steps* to be done for the doctor and for the patient.
1. The doctor *customizes* a training program for his patient based on the *target heart rate*, which he then *uploads* on the memory card or directly into the training device via internet.
2. The patient *plugs* the memory card into the training device (or connects to the Internet), puts on a heart rate controller and has only *a few indications* to follow on the LCD screen (e.g. begin cycling, speed up or down, session is finished). The training device *sets automatically the load* according to the training profile and the target heart rate and *uploads back* the sessions parameters on the memory card.
3. After a series of sessions the patient returns to the doctor that can now download the information (on the card or via the Internet). This *monitoring loop* will help the doctor to check if the scheduled program has been followed correctly and to adjust the following sessions according to the evolution of the medical parameters of the patient and the medical knowledge in the field of cardiovascular prevention by physical exercise.

## Description

### 1. CONTACT

Cardiology Medical Center
Willy KOSTUCKI, M.D.
Avenue Télémaque 32
1190 Brussels
BELGIUM
TEL # 32 475 494 454
TEL # 32 2 343 95 75
FAX # 32 2 343 79 80
willy.kostucki@chello.be

### 2. FIELDS OF INVENTION

THE PRESENT INVENTION RELATES TO THE FIELDS OF:
1. EXERCISE DEVICES
2. CARDIOVASCULAR REHABILITATION
3. CARDIOVASCULAR DISEASE
4 MEDICAL EQUIPMENT
5. RECORDING, COMMUNICATION, OR INFORMATION RETRIEVAL EQUIPMENT

### 3. INTRODUCTION

Dr Willy KOSTUCKI, MD, Cardiologist, designed a medical application in the field of cardiovascular rehabilitation and called it **EXERCISE MANAGER PROGRAM** or **EMP**.

The theoretical concept has been deposit in a notarized document, with the Notary Chantal LOCHE, **on January 23,2001.** This document also got an apostil for foreign use according to the Convention of La Haye, October 5, 1961. This bilingual document is entitled "LINKING DEVICE BETWEEN THE PHYSICIAN AND THE TRAINING MATERIAL: AN INNOVATION". This document is part ofto the present patent application and annexed to it.

The present patent application relates to the virtual **PROTOTYPE** of the innovation described in this document.

The innovation can be summarized as follows.

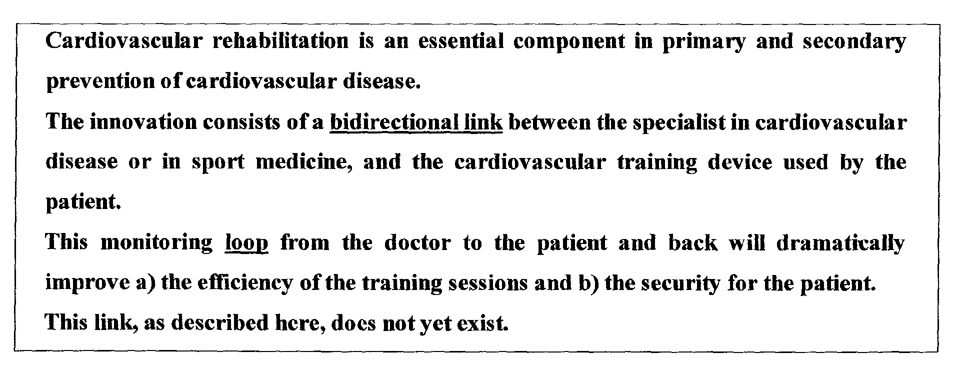

### OVERVIEW OF CURRENTLY AVAILABLE EQUIPMENT

Some excellent equipment is currently available, such as high-quality bicycles or treadmills, and reliable heart-rate monitors, so that stress levels and heart rates can be easily monitored.

### WHAT IS LACKING?

What is currently lacking is the bidirectional link between the physician (usually the cardiologist) and the patient's training equipment.

### INNOVATION

The innovation consists of a BIDIRECTIONAL LINK between the specialist in cardiovascular disease, or in sport medicine, and the cardiovascular training device used by the patient. This monitoring loop, from the doctor to the patient and back, will dramatically improve the efficiency of the training and the security for the patient.

This link has the following aims:
1. To achieve the best possible results when training a patient according to the program prepared by his physician.
2. To monitor the training sessions in order to check if the scheduled program has been followed correctly and to adjust the following sessions according to the evolution (improvement or worsening) of the medical parameters of the patient and the medical knowledge in the field of cardiovascular prevention by exercise.

The link will use either a memory card-like technology or the Internet technology.

Before entering the technical details of the PROTOTYPE, some basic knowledge in cardiovascular rehabilitation is needed to fully understand the innovation.

### EXERCISE TRAINING IN PRIMARY AND SECONDARY PREVENTION FOR CORONARY HEART DISEASE. DATA FROM THE LITERATURE

Primary prevention refers to the prevention of a disease in a normal population (supposed not having the disease).

Secondary prevention refers to patients with proven cardiovascular disease, e.g. patients after recent cardiac event, after bypass surgery, during heart failure, after valvular replacement. Secondary prevention is the prevention of worsening of a disease or preventing new event to occur.

It has been clearly established that exercise and fitness reduce Coronary Heart Disease and that improving fitness is as powerful as changing other major cardiovascular risk factors. There is a strong dose-response relationship. This is true for both sexes and recently demonstrated for all ages, this means that exercise training is very important for the elderly, making this huge population a particular target for cardiovascular rehabilitation.

### THE PARADOX THAT LEAD TO THE DEVELOPMENT OF THE EXERCISE MANAGER PROGRAM (EMP)

### HIGHER RISK PATIENTS REQUIRE GREATER LEVEL OF TRAINING.

It also has been demonstrated that occupational and leisure activity has some beneficial effect in the normal population. But data from the medical literature also show a paradox: *higher risk patients require greater level of training* in order to get benefit from cardiovascular rehabilitation. But in practice the data also show that those patients are usually away from the rehabilitation programs.

What are the reasons for that?

### 1. LACK OF CONFIDENCE FROM THE PATIENT

Those patients are physically limited, by their heart status and sometime by their co morbidities. They also are psychologically hearted and *fear exercise training*.

What is indeed mandatory to start with is a simple tool that gives the patient a high level of **confidence** while training.
*This is where the EXERCISE MANAGER PROGRAM (EMP) started.*

### 2. LACK OF CONFIDENCE FROM THE DOCTOR

A strong factor for succeeding in training those patients is the primary care physician's understanding and his confidence in the safety of exercise training. This confidence can tremendously be enhanced when the physician will be able to set by himself a *customized program* for his patient and to clearly and easily *monitor the training sessions.* The doctor will be able to adjust the training program according to the evolution (improvement or worsening) of medical parameters of the patient and to the evolution of the medical knowledge in the field of cardiovascular prevention by physical exercise.

Such a system is currently lacking on the market, to the best of our knowledge.
*EXERCISE MANAGER PROGRAM (EMP) fills this gap.*

### 3. COMPLIANCE OF THE PATIENT

The patient's compliance represents presently the other obstacle to a winning cardiovascular rehabilitation.

The revalidation of cardiac patients is almost exclusively performed inside hospitals. But available resources in hospitals are limited on the one hand, and, on the other hand, patients who are returning to active life may not always be able or willing to conform to strict schedules. Time constraint to in-hospital programmes is a major factor for the low patient's compliance. Moreover, those still active patients want to remain independent from hospital structures. This leads to a major under-consumption of cardiac, vascular and pulmonary revalidation, with serious consequences both on public health and personal prognosis.
*EXERCISE MANAGER PROGRAM (EMP) answers this issue.*

### 4. INADEQUATE SETTING OF THE TRAINING DEVICE

As for the patients who are looking after themselves to train at home or at the gym, sometime with their doctor's push, they will often inadequately train. This is because they either choose a training level that is too low because of apprehension, or because they set themselves at an overly high training level, which could prove dangerous for their health. Also the settings ofthe devices are often too elaborated for most patients: too complicated on the one hand and not fully adapted on the other hand.
*EXERCISE MANAGER PROGRAM (EMP) dramatically simplifies the procedure of the setting on a "plug and play" basis.*

### 5. NO STRICTLY CUSTOMIZED PROGRAM AVAILABLE

The training structure may also be inadequate (interval training, for example, is not feasible without a device that has been programmed for).
*EXERCISE MANAGER PROGRAM (EMP) easily controls those parameters.*

### PRINCIPLES OF CARDIOVASCULAR AND PULMONARY REVALIDATION

Cardiovascular and pulmonary revalidation is based on a training mode called *endurance*, in opposition of the *isometric* mode. In the *endurance* mode the goal is to maintain a target heart rate during a determined time-span. The typical training tool for cardiovascular training is the training device, i.e. the static bicycle in Europe, or the treadmill more used in the US, which is coupled to a heart-rate monitor, an indispensable device that makes it possible for the patient to control his heart rate throughout the effort phases.

After a complete medical evaluation, taking into account a series of parameters, which it would be too long to mention here, the cardiologist is able to propose a tailor-made training program to his patient. Once this evaluation has been performed, the cardiac, vascular or pulmonary patient can be provided with a very precise training program.

### 4. DESCRIPTION

| **TABLE OF CONTENTS** | | | | |
|---|---|---|---|---|
| | | | | |
| **TABLE OF CONTENTS** | | | | **11** |
| | | | | |
| **CONTACT** | | | | **12** |
| | | | | |
| **BACKGROUND AND QUICK OVERVIEW** | | | | **13** |
| | | | | |
| **OBJECTIVE** | | | | **15** |
| | | | | |
| **OVERVIEW AND GENERAL DESCRIPTION** | | | | **16** |
| | PART I: OVERVIEW | | | **17** |
| | PART II: GENERAL DESCRIPTION | | | **17** |
| | PART III :FUTURE DEVELOPMENT INTERNET COMMUNICATION | | | **19** |
| | | | | |
| **DATA DESCRIPTION** | | | | **21** |
| | PART I :DESKTOP DATABASES DESCRIPTION | | | **22** |
| | | 1. DB1 :DESKTOP DATABASE | | **22** |
| | | 2. DB2 :PATIENT DATA | | **23** |
| | | 3.DB3 :TRAINING DATA | | **24** |
| | | 4.DB4:SESSION DATA | | **26** |
| | | | 1. SESSION IDENTIFICATION | **26** |
| | | | 2.TRAINING SESSIONS PARAMETERS | **26** |
| | PART II: MEMORY CARD DATABASES DESCRIPTION | | | **28** |
| | | 1.DATABASE A:DATA RELATED TO TRAINING SET-UP | | **28** |
| | | 2.DATABASE B:DATA RELATED TO THEPERFORMED SESSIONS | | **29** |
| | PART III:MEMORY SIZE ON THE MEMORY CARD | | | **30** |
| | | | | |
| **THE PROTOTYPE** | | | | **31** |
| | **PART I: EMP-DESKTOP** | | | **32** |
| | | MAIN INTERFACE | | **32** |
| | | PATIENT SELECTOR | | **33** |
| | | TRAINING SELECTOR | | **33** |
| | | TRAINING EDITOR | | **34** |
| | **PART II:EMP-SIMULATOR** | | | **36** |
| | | | | |
| **SOURCE CODE** | | | | **38** |
| | | | | |
| **CLAIMS** | | | | **39** |
| | | | | |
| **FIGURES** | | | | **40** |

### CONTACT

Cardiology Medical Center
Willy KOSTUCKI, M.D.
Avenue Télémaque 32
1190 Brussels
BELGIUM
TEL # 32 475 494 454
TEL # 32 2 343 95 75
FAX # 32 2 343 79 80
willy.kostucki@chello.be

### BACKGROUND AND QUICK OVERVIEW

Dr Willy KOSTUCKI, MD, Cardiologist, designed a medical application in the field of cardiovascular rehabilitation, in the beginning of 2000.

The innovation can be summarized as follows.

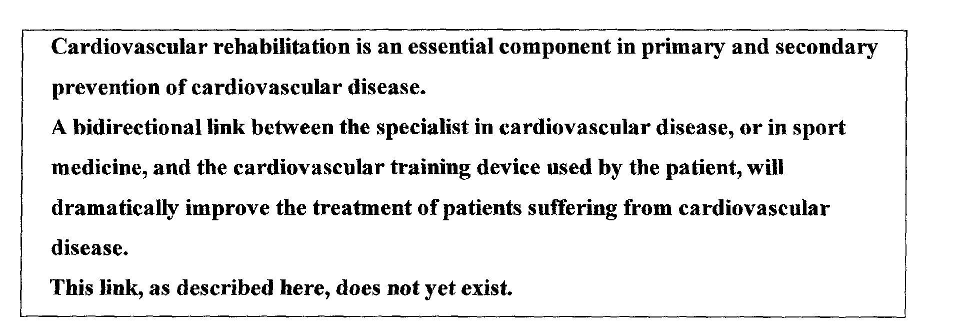

A **memory card** materializes this link. This **memory card** makes a link between two programs, one program on the doctor's computer desktop and the second program into the training device itself. This link has the following aims:
1.To **achieve the best possible results** when training a patient according to the program prepared by his doctor.
   The key parameter that sets the parameters of the training device is the **heart rate** of the patient. The user friendliness is based on a **plug and play** mode without complex intervention ofthe patient or the doctor.
2.To **monitor** the training sessions in order to check if the scheduled program has been followed correctly and to adjust the following sessions according to the evolution (improvement or worsening) of the medical parameters of the patient and the medical knowledge in the field of cardiovascular prevention by physical exercise.
   The key point of this part of the system is the possibility given to the doctor in charge to **customize** a training profile (steady state training, interval training...).

Dr Willy KOSTUCKI called the all system **EXERCISE MANAGER PROGRAM**, or **EMP**.

The ultimate goal of Dr KOSTUCKI is to market his application.

In order to develop a prototype for this application Dr KOSTUCKI contacted the Information and Decision Systems Department of Faculty of Applied Sciences (Free University of Brussels) by October 2001.

The present document is the result of several months of creative and fruitful collaboration between Dr KOSTUCKI and both Professor Philippe VAN HAM and Olivier DEBEIR, from the Decision Systems Department of Faculty of Applied Sciences (Free University of Brussels).

The Exercise Manager Program is exclusively the property of Dr Willy KOSTUCKI.

### OBJECTIVE

This document describes the software prototype designed by Dr Willy KOSTUCKI and implemented in collaboration with the Decision Systems Department of Faculty of Applied Sciences (Free University of Brussels).

**EMP** refers to **Exercise Manager Program** throughout the present document.

The aim of the prototype is to show how the Exercise Program Manager is practically operating.

This software prototype is intended to be the basis of the platform for a future commercial application, according to Dr KOSTUCKI's goals. Indeed this prototype is not only a series of theoretical drawings as one can find on various Internet sites dedicated to patent registration. All the programs in the system, from the doctor's desktop to the patient training site, are open. All the steps are written and the source codes and binary files are available. A CD with an install.exe for both the desktop and the simulator programs (respectively EMP Desktop and EMP Simulator) is annexed to this document.

The prototype illustrates the complete application from the doctor's desktop to the patient training site. 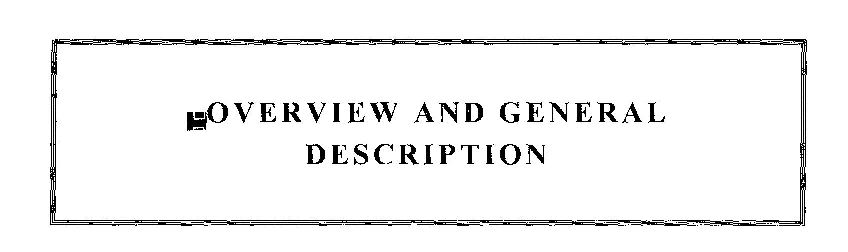
**PART I: OVERVIEW**
**PART II: GENERAL DESCRIPTION**
**PART III: FUTURE DEVELOPMENT: INTERNET COMMUNICATION**

### PART I: OVERVIEW

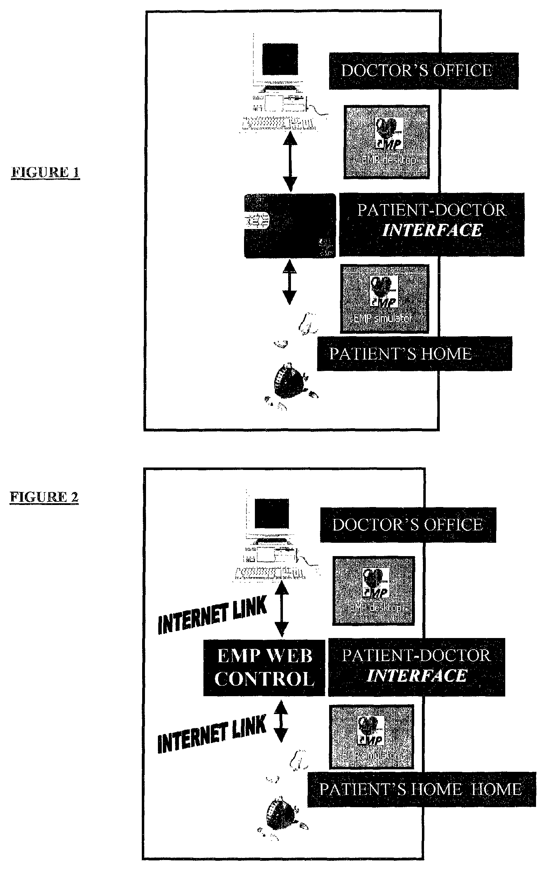

### FIGURE 1- SEE ANNEX

### PART II: GENERAL DESCRIPTION

Figure 1 (Overview) shows the general organization.
**EMP** refers to **Exercise Manager Program.**
The link between the doctor's office and the patient's training site is materialized by a personal **memory card** such as a flash card, at an average size of a credit card.

### CHRONOLOGICAL STEPS

1. The memory card is programmed by the doctor in his office and handed over to the patient.
2. Upon returning home the patient then inserts the card in his training device (e.g. bicycle or treadmill), which is then automatically programmed. Thus the patient must only perform a limited number of manipulations and, most importantly, no technical programming at all (**plug and play** basis). In particular, the patient does not have to introduce parameters like age, sex, and weight, level of exercise, etc, as is recommended on most available home training devices.
3. When the patient performs his training routine, several data are captured by the training device and inscribed on the card. For example: duration of each training session, heart rate distribution (maximal, mean, heart rate-time distribution), the workloads (maximal, mean, heart-rate time-diagram), and, possibly, other parameters to be added by the specialist physician.
4. On the patient's next visit to his doctor, he returns his memory card to the physician.
5. The doctor then reads the memory card via his computer using a available reading module, or via an independent module (a cradle). Then, after only a few mouse-clicks, the doctor is now able to have a complete **overview of his patient's training sessions.** He can now **correct** what needs correcting and easily reprograms the memory card for the following training period and hands it over back to the patient. The programmed parameters can always be **adapted** according to the patient's condition and the training **profiles** can always be adapted to the evolution of the knowledge in the field and scientific recommendations.
6. And a new cycle begins again.

The prototype is made of two main programs:
1. A program called "EMP-Desktop": this is the application that will be used by the doctor.
2. A program called "EMP-Simulator": this is the application that should theoretically be implemented on a training device. In this case it is a simulator of a patient and a training device.

In order to show the feasibility of the application, both programs will communicate with each other by means of simple text files, in order to ensure that the electronic card, in the final application, will be properly able to carry out the amount of data.

### PART III: FUTURE DEVELOPMENT: INTERNET COMMUNICATION

### Figure 2 - see annex

Figure 2 shows that the physical link between the doctor and the training device can be replaced by an Internet link, either directly from the doctor's computer to the training device or via an intermediate web site.

The cornerstone is always to insure the **bidirectional** feature of the system: the doctor must always be able to control the parameters of the training device and the data recorded from the sessions performed by the patient must reached the doctor's computer.

This data can reached its target (doctor or training device) either by online monitoring or by delayed monitoring.
1. In case of a direct connection from the doctor's office to the training device, various modalities can be proposed according to both sides desires:
   - **Online monitoring:** this will not be recommended in general but might be useful for special difficult cases or to initiate a rehabilitation program in medically difficult or very anxious patients.
   - **Delayed monitoring:** the data of the performed sessions will be stored directly into the **EMP-Desktop Program** developed in this document, directly in the patient's personal file, giving the opportunity for the doctor to analyze the data at his best convenience and to correct the parameters immediately from his computer into the training device, via the internet connection (the internet module for this program can be one of the many commercially available modules performing this task).
2. In case of a connection via an Internet web site, the data recorded from the sessions performed by the patient are stored on a site called **EMP WEB CONTROL CENTER.** A series of **filters** will help the manager of this control web center to track the sessions not performed properly or showing medical or technical problems. The manager can then contact, by either way possible, the doctor in charge to modify the program or to take any medical decision. In case nothing special is to be reported, which will be the vast majority of cases, the data of the performed sessions are immediately **forwarded** to the doctor in charge, directly in the patient's personal file of the **EMP-Desktop Program** developed in this document. The details of this web control program will be part of another document.

Both **Internet connection** AND **memory card** can work together. One modality does not exclude the other. This still gives the patient the opportunity to physically bring back his memory card to the doctor at the next medical visit even if the data will already be transferred via the Internet connection.

The Internet module is scheduled for development in the next future. 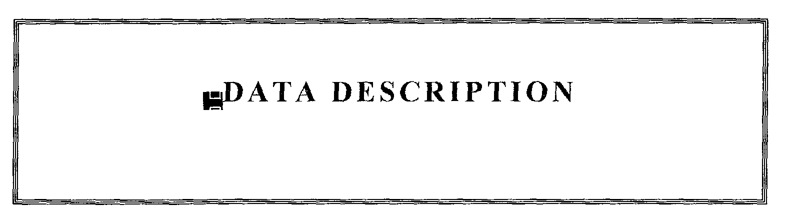
**PART I: DESKTOP DATABASES DESCRIPTION**
**PART II: MEMORY CARD DATABASES DESCRIPTION**
**PART III: MEMORY SIZE ON THE MEMORY CARD**

### PART I: DESKTOP DATABASES DESCRIPTION

Four databases are currently involved in the presently described application.

Those databases are located on the computer at the doctor's office.
1. DB1: desktop database
2. DB2: patient data
3. DB3: training data
4. DB4: session data

### 1. DB1: DESKTOP DATABASE

The doctor's desktop database contains all the information that is needed for the follow-up of the patients.
- A complete history of the sessions for one single patient: the dates the sessions were performed, the time spent on the bike per session, data about mean and maximal heart rate, type or profile of the stress, etc.
- A statistical database for cross-comparison between patients.
- The database of the profiles of the sessions. The profile can be customized according the physician desires, e.g. steady state profile, interval training, and much more.

The data are organized in a hierarchy, like the one shown in the next figure:

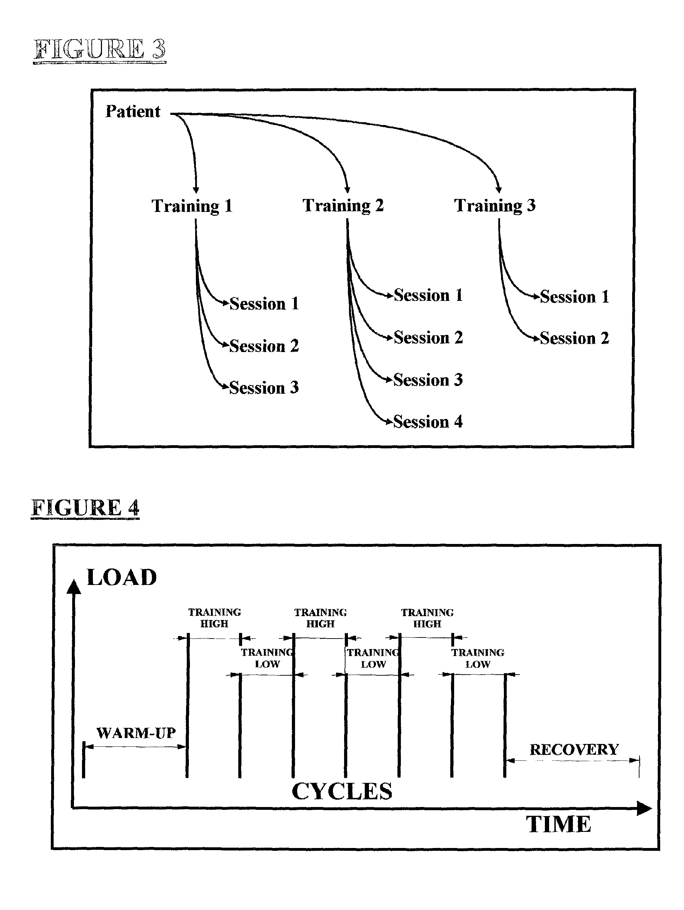

### Figure 3 - see annex

### 2. DB2: PATIENT DATA

The patient data are arranged according to the following fields:
- Patient UID (unique identifier assigned to each new patient)
- Patient Name (String)
- Patient Social Number (integer)
- Patient Sex (Boolean)
- Patient Birth Date (Date)
- Patient Address (String)
- Patient Telephone (String)
- Patient e-mail (String)
- Other data can be implemented according to the country's legislation or the rules in use in each place.

### 3. DB3: TRAINING DATA

The data related to a training session include:
- Total training duration
- Target heart rates at different intervals
- Time of the warming-up period
- Time of the recovery period
- Type or profile of the exercise, i.e. steady state profile or interval training
- Number of cycles (1 for a steady state training and > 1 for interval training)
- Target heart rates for each interval and the regulation parameters that will drive to the training device

### FIGURE 4- see annex

Figure 4 is an example of a typical session with an interval type profile. There is a customized warm-**up period**, in this case two level of intensity (**training high and low**), 6 **cycles**, and a **recovery period**.

The training parameters are:
- Training Profile UID (**U**nique **Id**entifier given for each new training profile) A training profile is unique and has its own UID. This means that the training profile can easily be duplicated for another patient. The duplication setting is optional. This option can save much time for the doctor when he designed a new session program for a new patient.
- Training Creation Date (Date)
   Creation date of the training profile.
- Training Profile Name (String)
   Name that allows the doctor to remember quickly the training profile.
- Number Of Cycles (Integer)
   Number of cycles during the training phase in a training session.
- Patient UID (integer)
   Patient for which this particular training profile is prescribed.
- Warm-up Target (Integer)
   Target Heart Rate for the Warm-up phase.
- Warm-up Alarm (Integer)
   Heart Rate upper limit for the Warm-up phase.
- Warm-up Speed Max (Integer)
   Upper limit for the bike rotation speed. If the limit is crossed, an alarm message will appear on the LCD display, to invite the user to speed down.
- Warm-up Speed Min (Integer)
   Lower limit for the bike rotation speed. If the limit is crossed, an alarm message will appear on the LCD display, to invite the user to speed up.
- Warm-up Time (Integer)
   Duration of the Warm-up phase.
- Warm-up Load Max (Integer)
   Upper limit of the load during the warm-up phase. It limits the maximum load the patient will have to face. This is mainly intended for security reasons.
- Warm-up P (Float)
   Proportional parameter of the bike regulator during the Warm-up phase.
- Warm-up I (Float)
   Integer parameter of the bike regulator during the Warm-up phase.
- Warm-up D (Float)
   Derivative parameter of the bike regulator during the Warm-up phase.

The same data are used for the other phases, i.e.:
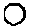 Training High
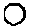 Training Low
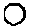 Recovery Phase

### 4. DB4: SESSION DATA

Sessions data are captured during the training sessions.

The first fields are related to the session identification. The other fields will be filled with data captured during the different phases of the training session.

### DATABASE ORGANIZATION:

**1. SESSION IDENTIFICATION**
2. TRAINING SESSIONS PARAMETERS

### 1. SESSION IDENTIFICATION

- Session Date (Date)
   Date the session is carried out by the patient
- Session Time (Time)
   Hour, min and sec when the session begun (HH/MM/SS)
- Session UID (unique identifier)
   Allows identifying each session unambiguously throughout the whole database
- Training UID (Integer)
   UID of the training description used for the training sessions
- Analyzing Date (Date)

Date when the card is read (i.e. when the data are downloaded by the physician when the patient comes back to his office).

### 2. TRAINING SESSIONS PARAMETERS

The following data are recorded during each phase of the training session:
- Time1 (integer)
   Duration of the training phase in second
- BPMbegin1 (float)
   Heart Rate at the beginning of the phase
- BPMend1 (float)
   Heart Rate at the end of the phase
- BPMmin1 (float)
   Minimum Heart Rate during the phase
- BPMmax1 (float)
   Maximum Heart Rate during the phase
- BPMsum1 (float)
   Cumulated Heart Rate during the phase (in order to obtain the Mean Heart Rate in Beats Per Minute ― BPM -, this number is to be divided by Time 1)
- Cminl (float)
   Minimum Load during the phase
- Cmax1 (float)
   Maximum Load during the phase
- Csum1 (float)
   Cumulated Load during the phase (to obtain the average Load, this number is to be divided by Time1)

As already mentioned, the same data are recorded for each phase of each session.

### PART II: MEMORY CARD DATABASES DESCRIPTION

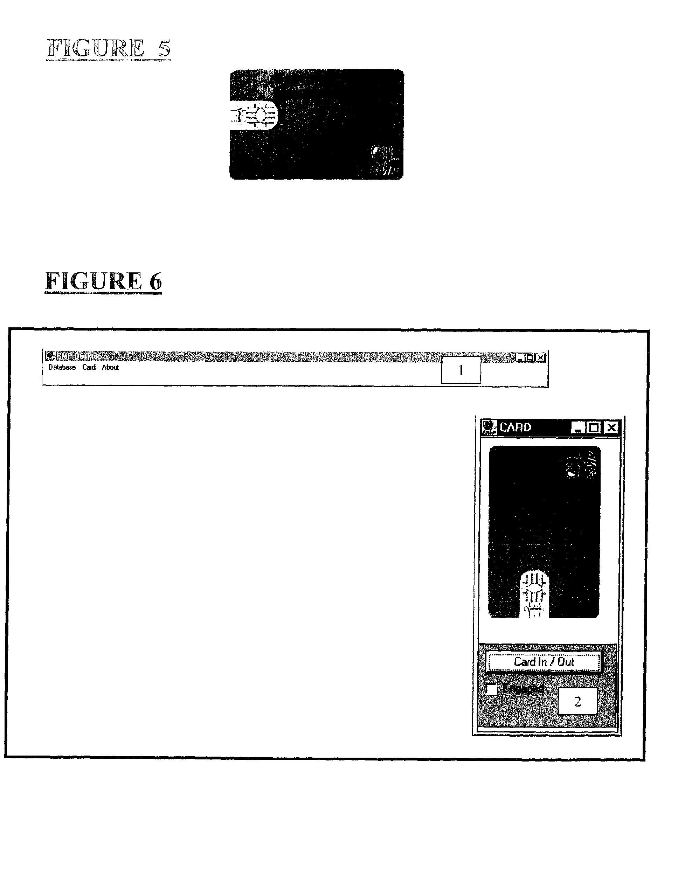

### FIGURE 5 - see annex

The memory card includes the following databases:
1. Database A: data related to the training set-up
2. Database B: data related to the performed sessions

### 1. DATABASE A: DATA RELATED TO THE TRAINING SET-UP

Here is a typical example of the card content for the training description. As far as the prototype is concerned, data are stored in a text file called card.txt. The string content is just put as comment. In the real application, only *numbers* will *be stored (e.g. 32bit floating point numbers*).

The aim here is just to illustrate how data could be recorded in a device like a simple I²C memory card.
21 *UID_patient*
37 *UID_training*
100 *Warm_up_target_BPM*
200 *Warm_up_alarm_BPM*
40 *Warm_up_speed_MIN*
60 *Warm_up_speed_MAX*
15 *Warm_up_time*
100 *Warm_up_load_MAX*
*-0.100000 Warm_up_P*
*-0.200000 Warm_up_I*
0.000000 *Warm_up_D*
150 *Training_HI_target_BPM*
200 *Training_HI_alarm_BPM*
40 *Training_HI_speed_MIN*
60 *Training_HI*_speed*_MAX*
10 *Training_HI_time*
200 *Training_HI_load_MAX*
- 0.400000 *Training_HI_P*
- 0.200000 *Training_HLI*_*I*
0.000000 *Training_HI_D*
100 *Training_LOW_target_BPM*
200 *Training_LOW_alarm_BPM*
40 *Training_LOW_speed_MIN*
60 *Training_LOW_speed_MAX*
10 *Training_LOW_time*
200 *Training_LOW_load_MAX*
- 0.400000 *Training_LOW_P*
*-0.200000 Training_LOW*_*I*
0.000000 *Training_LOW_D*
*1 Training_cycles*
80 *Recovery_target_BPM*
200 *Recovery_alarm_BPM*
40 *Recovery_speed_MIN*
60 *Recovery_speed_MAX*
15 Recovery_time
100 *Recovery_load_MAX*
-*0.100000 Recovery_P*
*-0.200000 Recovery_I*
0.000000 *Recovery_D*

### 2. DATABASE B: DATA RELATED TO THE PERFORMED SESSIONS

Here is a typical example of the card content for the performed sessions. As far as the prototype is concerned, data are stored in a text file called card.txt. The string content is just put as comment. *In the real application, only numbers will be stored (e.g.32bit floating point numbers*). Also date and time data will be converted into single numbers.
*8*/*01*/*02 11:52:44 16.000000 20.661249 97.687531 20.661249 97.687531 1133.376709...*
*8*/*01*/*02 11:53:59 16.000000* *55.718678* *104.969193 55.718678 104.969193 1350.251953...*

### PART III: MEMORY SIZE ON THE MEMORY CARD

The data of both the training sessions set-up and data related to the performed sessions will have to fit in the memory of the card. On the card, the amount of memory used should be approximately 168 bytes for the training data and 128 bytes for each session data. Typical I²C memories are presently 32 or 64 KB large. Therefore, in a 64kb memory (8kbytes), it will be possible to store 1 training description (168 bytes) and 61 session records (128 bytes each).

By the time the product will be launched into the market the size of the memory cards will certainly increase, making it possible to store much more data (recordings and settings). 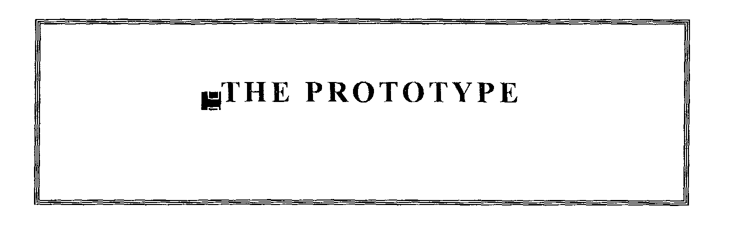
**PART I: EMP - DESKTOP**
**PART II: EMP - SIMULATOR**

### PART I: EMP - DESKTOP

The first part of the prototype is the application program on the doctor's desktop, called EMP-Desktop.

This program is aimed to manage the patient database and the database of the training profiles. This program also deals with the data containing on the memory card that are downloaded from the memory card after a set of training sessions are performed by the patient, i.e. when the patient returns to the doctor's office.

This program allows export of all the data into a standard text file format in order to be used by any spreadsheet-like program for future development (graphs, statistics...).

### MAIN INTERFACE

### FIGURE 6- see annex

This window represents the main interface. The two items are.
1/ the menu bar 1
2/ A virtual cradle with the card to be inserted or remove 2

*1*/ *The menu bar* gives access to the **Patient selector** (see after) and **Card Check** functionality, i.e. card validity (this function is purely virtual for this prototype but should be functional in the real application), and card reading.
2/ *The virtual cradle* shows how the card will be inserted/extracted into/from the cradle.

The card functionality will be operational only when the card is inserted.

### PATIENT SELECTOR

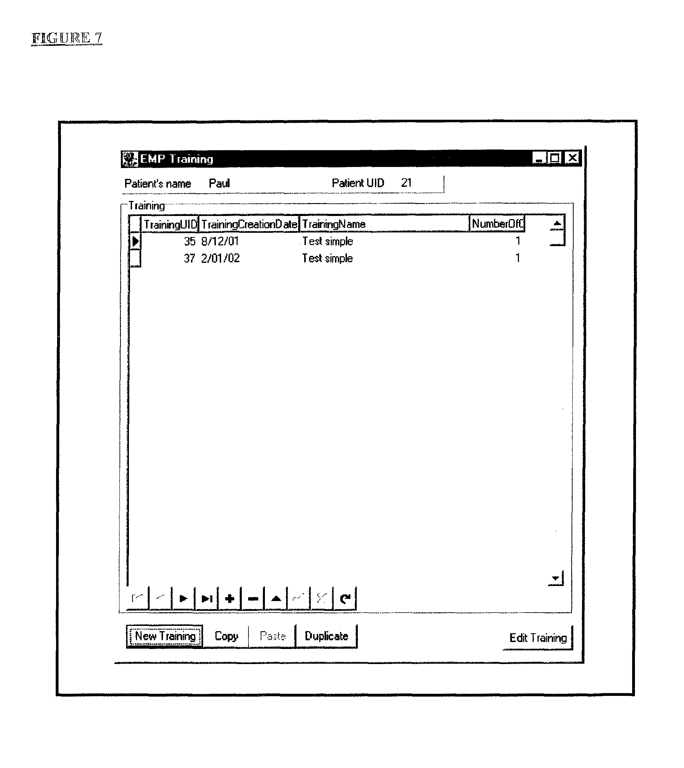

### FIGURE 7-see annex

This window allows browsing into the database ofthe patients.

From this window the doctor can add a new patient or modify the data of an existing patient.

The **Training** button (lower right comer) launches the **Training Selector** (see below).

### TRAINING SELECTOR

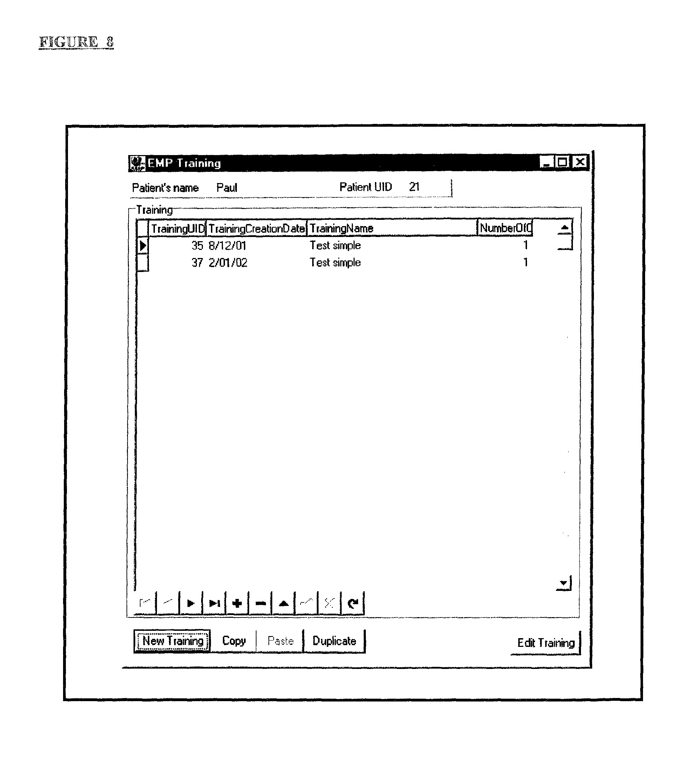

### FIGURE 8 - SEE ANNEX

This window is used to add a new training profile into a given patient's folder. Three methods can be used:
1.Add a new training profile from scratch **(+ button)**
2.Duplicate an existing training profile from the patient folder **(Duplicate button)**
3.Copy a training profile from an other patient folder (**Copy** and **Paste button**)

To edit a selected training, use the **Edit Training button**. Before editing a new training, be sure it is effectively recorded (the symbol on the extreme left column should contain an **arrow**, meaning this training profile is selected).

### TRAINING EDITOR

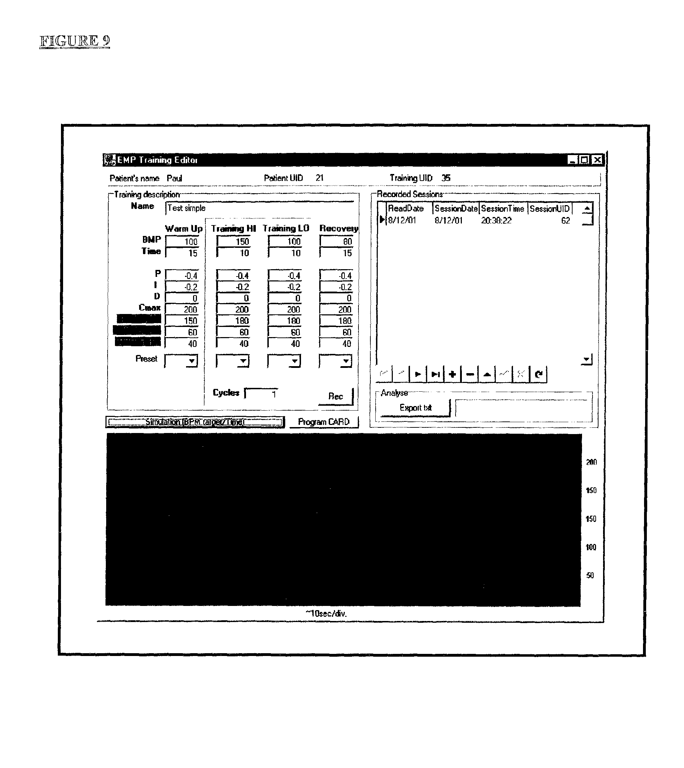

### FIGURE 9- see annex

To access to this window simply click on the **Edit Training** button from the previous **Training Selector** screen.

From this window the doctor can create new trainings profile.
1. The user introduces manually each parameters of his newly designed training profile, making **customization** of the training profiles a key point in the system.
2.The user then clicks on a pop-up menu **(Preset)** and all the parameters of the column are set to a predefined value. These values can be trimmed in the *presets.ini* file.

After the training parameters are set, it is possible to have a quick visualization of the training profile, exactly as it will operate with a patient, by clicking the **Simulation button**. By clicking on the **Program Card button**, the present training profile is uploaded into the memory card that has just been plugged into the cradle.

All needed information is copied, among other the Patient UID, the Training UID and the training parameters.

In case data from previous sessions are still stored on the memory card, the parameters of those sessions are not erased (a warning message will tell you some data exist on the memory card).

If the training profile needs to be modified, a new copy of it must be made.

The Export.txt button allows exporting the data of the training session in a standard text file (export.txt) in order to be imported in other programs.

The next illustrations show the different control tabs:

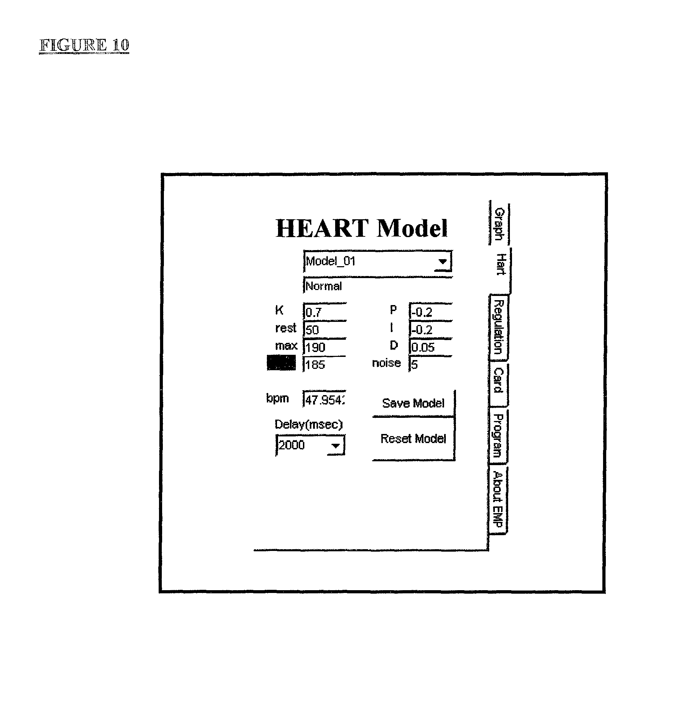

### FIGURE 10- see annex

### Heart Model Parameters

**Model 01 to 05:** one interesting feature of the system is the possibility to preset up to five different profiles (in a pop-up menu). The name of the profile in use is written below. This feature is especially intended to create profiles, by setting the correct P, I and D, that can be directly related to special conditions such as patients with severe deconditionning, patients with heart rate disease, with ventricular dysfunction, ...

**Delay** is the model update frequency.

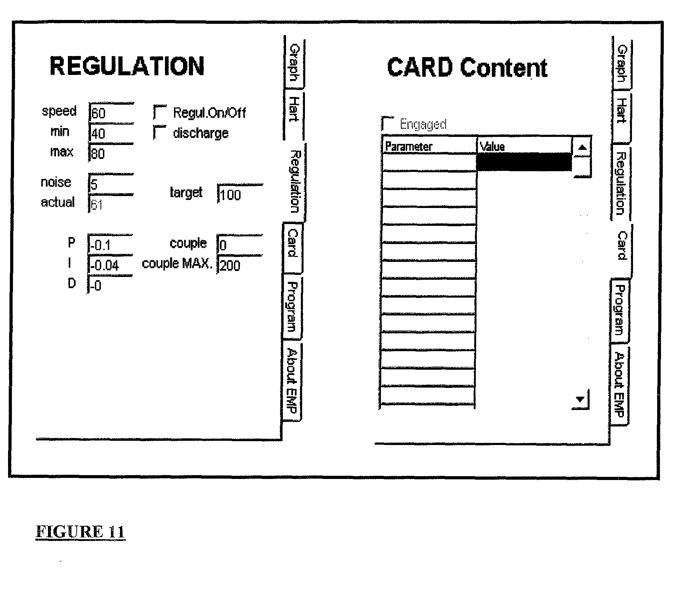

### FIGURE 11-see annex

**Regulation:** this tab permits to test manually the regulation settings.

The Card Content displays the content of the inserted card (if the card is not inserted, the fields are empty).

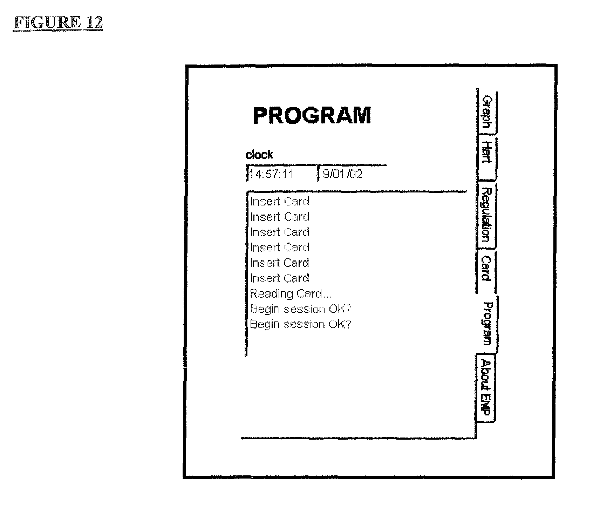

### FIGURE 12-see annex

The **Program** page logs the current training program status.

### PART II: EMP - SIMULATOR

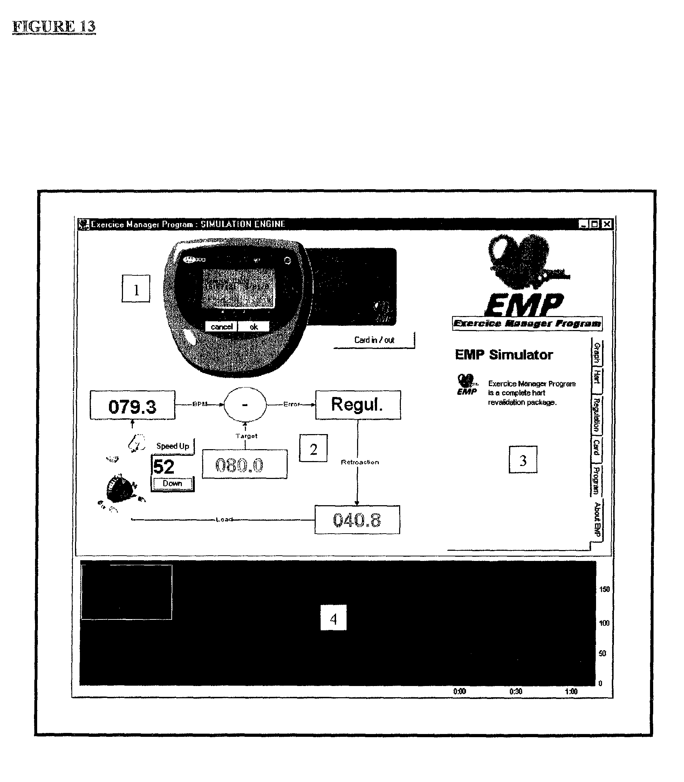

### FIGURE 13 -see annex

The simulator is operational in just one window.

This window is divided into four sub-screens:
1 The upper left part of the figure is the bike LCD screen as seen by the patient.
2 The left center of the figure is the regulation loop with the different dynamically refreshed values. This allows quickly seeing and appreciating the interaction between the main parameters: rotation speed and **Target Heart Rate.**
   When rotation is too slow or too fast, a warning message appears on the screen.
   The simulator allows increasing or decreasing the rotation speed in order to test the retroaction of the system.
3 At the right center of the illustration is the control panel (see below) that allows following the dynamic relations between all the parameters, as set on the memory card at the doctor's desktop (see previous section).
4 At the bottom of the frame, the dynamic graph of the different parameters, the most important being the Target Heart Rate and the Actual Heart Rate:
   1. Heart rate (BPM)
   2. Target heart rate (BPM)
   3. Bike rotation speed (RPM)
   4. Work load (W)

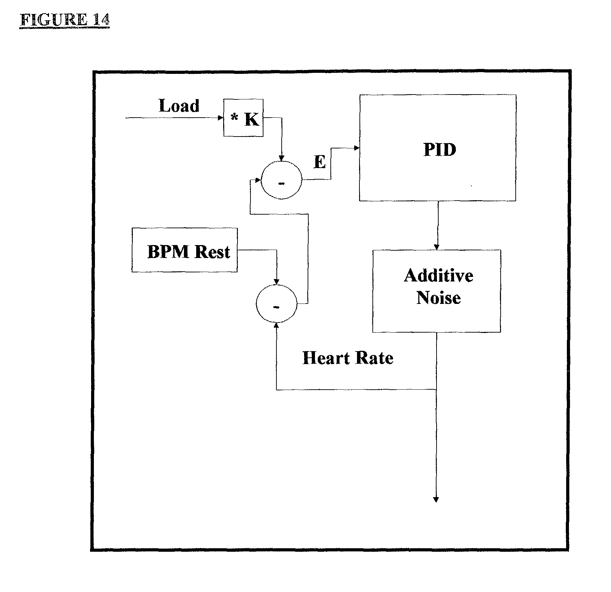

### FIGURE 14-see annex

The simulator uses the following Heart Model:

The figure shows how the new heart rate of the model is computed. The *K factor* is the general impact of the load on the patient heart: if the patient is fit and able to develop a high load, K will be low, if the patient is under trained and is only able to develop a low load, K will be high. *BMP* Rest is the heart rate at rest for the patient (without any load). The PID regulator gives the response to a variation of load. Depending of its internal parameters (P, I and D), the regulator will give a fast or a slow reaction to the load changes. An additive noise is added to the heart rate to simulate the variations of the heart rate measurements that may occur in a real application, and the chaotic behavior of some hearts in various pathological situations. The developed prototype allows defining up to 5 different heart models (sets of heart parameters) in order to test different types of patient according to their clinical situation.

The models are saved in the *heart_ models.ini* file.

The simulator uses the following Regulation Model:

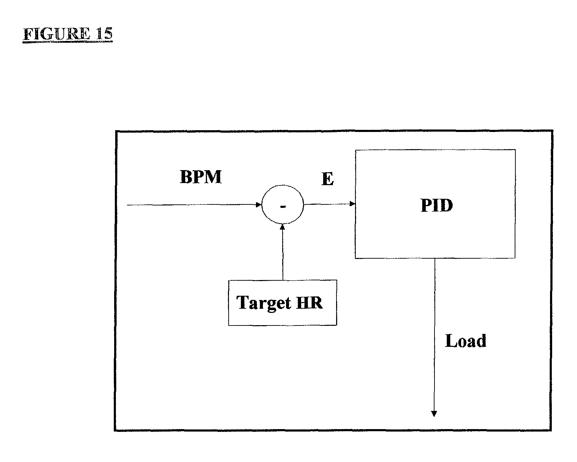

### FIGURE 15 - see annex

The figure 15 shows the regulation loop on the side ofthe *bike* or another training device. The input is the **HEART RATE**, and the output is the **workload**. The **PID** coefficients are the parameters of the regulation. These parameters are stored on the card, for each of the four phases of the training session. By changing the regulation parameters, it is possible to conceive a training device that respond fast or slowly in order to reach the target heart rates.

The prototype simulate the following training program:

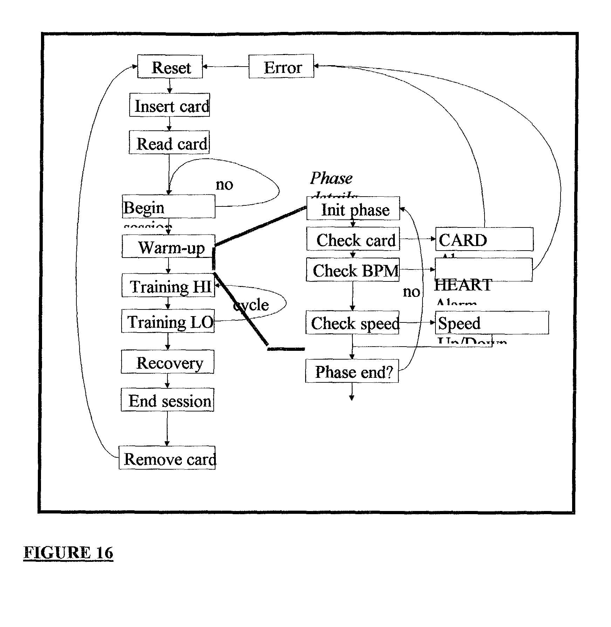

### FIGURE 16-see annex

The figure 16 shows how the micro-controller embedded into the training device should control the training program session. The regulation loop (see before) is running simultaneously.

Each of the training phases (left column) is composed of the **Phase details** (see center of the figure).

### SOURCE CODE

All sources and binary files are available on a CD.

The CD provided with this patent application is a .EXE program that shows all the functionalities of both described program as described in this document.

## Claims

1. The global claim is a cardiovascular rehabilitation s*ystem* consisting of a *program* on the desktop of the doctor's computer + a *program* into a training device + a *physical link* using either a memory card technology or the Internet technology + a functioning virtual *prototype.*

2. The *system.*
A cardiovascular rehabilitation *system* for improving the efficiency and safety of the training sessions performed by a patient according to the program prepared by his doctor. This system is a **monitoring loop system** from the doctor to the training device used by the patient and back. It is a **bidirectional link** which is intended a) to achieve the best possible results when training a patient, b) to monitor the training sessions in order to check if the scheduled program has been followed correctly and c) to adjust to the evolution (improvement or worsening) of the medical parameters of the patient and to adjust the following sessions according to the evolution of the medical knowledge in the field of cardiovascular prevention by exercise. The link is materialized by a memory card, which makes the physical link between the doctor's office and the training device used by the patient. The link can also use the Internet technology instead or in addition to the memory card. The whole system is thus a *program* on the desktop of the physician's computer + a *program* into the training device + a *physical* link using either a memory card technology or the Internet technology + a functioning virtual prototype.

3. The *memory card*.
The memory card includes the following databases:
Database A: data related to the training set-up.
Database B: data related to the performed sessions.
Database A: data related to the training set-up:
As far as the prototype is concerned, data are stored in a text file called card.txt. The string content is just put as comment. In the real application, only numbers will be stored (e.g.32bit floating point numbers).
Data stored on the memory card include at least (unlimited list): UID (user identifier) of the patient, UID (user identifier) of the training, warm up target heart rate, warm up alarm heart rate, warm up speed lower limit, warm up speed upper limit, warm up time, warm up maximal load, warm up P, warm up I, warm up D, training target heart rate upper limit. The profile of the exercise is determined by the following parameters: a) HI meaning the highest target heart rate part of the cycle b) LO meaning lowest target heart rate part of a cycle training and c) the number of cycles. Thus if a steady state profile is chosen the number of cycle will be 1 with only HI programmed. If an interval training is programmed the number of cycles will be >1, with HI and LO programmed separately. The additional parameters on the memory card are: HI alarm upper limit of heart rate, HI training speed minimum, HI training speed maximum, HI training time, HI training load maximum, HI training P, HI training I, HI training D, LO training target heart rate, LO training alarm heart rate, LO training speed minimum, LO training speed maximum, LO training time, LO training load maximum, LO training P, LO training I, LO training D, number of training cycles, recovery target heart rate, recovery alarm heart rate, recovery speed minimum, recovery speed maximum, recovery time, recovery load maximum, recovery P, recovery I, recovery D.
Database B: data related to the performed sessions.
As far as the prototype is concerned, data are stored in a text file called card.txt. The string content is just put as comment. In the real application, only numbers will be stored (e.g.32bit floating point numbers). Also date and time data will be converted into single numbers.
The following data at least (unlimited list) are recorded during each phase of the training session:
Time phase 1 (integer), Duration of the training phase 1 in second, heart rate begin session (float), heart rate at the beginning of the phase, heart rate end phase 1 (float), heart rate at the end of the phase 1, heart rate minimum phase 1 (float), minimum heart rate during the phase 1, heart rate maximum phase 1 (float), maximum heart rate during the phase 1, heart rate sum phase 1 (float), cumulated heart rate during the phase (in order to obtain the mean heart rate in Beats Per Minute ― BPM -, this number is to be divided by time phase 1), minimum load during the phase 1, maximum load during the phase 1, cumulated load during the phase (to obtain the average load, this number is to be divided by time 1).
The same data are recorded for each phase of each session.
In practice the card is used as follows.
The memory card is programmed by the doctor in his office and handed over to the patient.
• The patient inserts the card in his training device (whatever training device), which is then automatically programmed. The training device is operational soon after the introduction of the memory card into the training device. The system works on a "plug and play" principle. A limited number of manipulations is needed and, most importantly, no technical programming at all is required. Contrariwise of what exists on the commercially available training devices, the patient does not have to introduce parameters like age, sex, and weight. Also the patient does not have to introduce the level of exercise, the duration of exercise, the profile of exercise, the duration of the warming up period, the duration of the recovery period. All those data are recorded onto the memory card.
• When the patient performs his training routine, several data are captured by the training device and inscribed on the card. For example: duration of each training session, heart rate distribution (maximal, mean, heart rate-time distribution), the workloads (maximal, mean, heart-rate time-diagram), and, possibly, other parameters to be added by the doctor. For more details, see above and see the virtual prototype.
• On the patient's next visit to his doctor, the patient returns his memory card to the doctor. Memory size on the memory card: the data of both the training sessions set-up and data related to the performed sessions will have to fit in the memory of the card. On the card, the amount of memory used should be approximately 168 bytes for the training data and 128 bytes for each session data. Typical I²C memories are presently 32 or 64 KB large. Therefore, in a 64kb memory (8kbytes), it will be possible to store 1 training description (168 bytes) and 61 session records (128 bytes each).
By the time the product will be launched into the market the size of the memory cards will certainly increase, making it possible to store much more data (recordings and settings).

4. *The program at the doctor's office.*
The doctor reads the memory card via his computer using a available reading module, or via an independent module (a cradle) and downloads the whole information on the memory card into a program (called in this prototype EXERCISE MANAGER PROGRAM - DESKTOP). A complete overview of the patient's training sessions is now available for the doctor. He can now correct what needs correcting and easily reprograms the memory card for the following training period and hands it over back to the patient. The programmed parameters can always be adapted according to the patient's condition and the training profiles can always be adapted to the evolution of the knowledge in the field and scientific recommendations.
And a new cycle begins again. The details of the program can be see in the prototype description annexed to this document.

5. *The program into the training device.*
The memory card is intended to drive the training device in order to reach several target heart rate settings (this is a basic concept in cardiovascular training) in the various phases of the training session (warm up period, 1 target heart rate in case of a steady state profile or several target heart rate settings in interval profiles, recovery period). The heart rate can be monitored using any commercially available system, provided the data (heart rate) can be computerized in order to be read by the training device. The physical and mathematical principles of the card driving the training device is best described in the prototype document annexed to the present claims in the section simulator.

6. *The simulator.*
The simulator simulates the responses of the training device to various stimuli and various behaviors of normal subjects or patients with various cardiac diseases or cardiovascular unusual conditions. This is what will be used in practice into the training device. The simulator uses the following heart model: the input is the **HEART RATE**, and the output is the **WORKLOAD**. The **PID** coefficients are the parameters of the regulation. These parameters are stored on the card, for each of the four phases of the training session. By changing the regulation parameters, it is possible to conceive a training device that respond fast or slowly in order to reach the target heart rates.
The K factor is the general impact of the load on the patient heart: if the patient is fit and able to develop a high load, K will be low, if the patient is under trained and is only able to develop a low load, K will be high. The PID regulator gives the response to a variation of load. Depending of its internal parameters (P, I and D), the regulator will give a fast or a slow reaction to the load changes. An additive noise is added to the heart rate to simulate the variations of the heart rate measurements that may occur in a real application, and the chaotic behavior of some hearts in various pathological situations. The developed prototype allows defining up to 5 different heart models (sets of heart parameters) in order to test different types of patient according to their clinical situation.

7. The *prototype.*
A virtual prototype shows precisely how the *system* works. A CD with installation procedure is annexed to the present document.

8. The end user of the training device mentioned in the previous claims, although first intended to be a patient suffering from cardiovascular disease, can also be any under trained person or anyone who desires to improve his fitness.

9. The training device ofthe previous claims can be any training device presently on the market or will be on the market. The basic principle is the 'target heart rate' and not the type of exercise performed.
The person permitted to program the memory card on the desktop of the computer as described in the previous claims, although described here to be a medical doctor, can be anyone else claiming competence to do so.

10. The link of claim 1, although described in this prototype as a memory card, can also use the Internet technology. The same information, in both directions, as described in claim 2 is transmitted via the Internet.
• In case of a direct connection from the doctor's office to the training device, various modalities can be proposed according to both side's desires:
Online monitoring: this will not be recommended in general but might be useful for special difficult cases or to initiate a rehabilitation program in medically difficult or very anxious patients.
Delayed monitoring: the data of the performed sessions will be stored directly into the program on the doctor's computer, directly in the patient's personal file, giving the opportunity for the doctor to analyze the data at his best convenience and to correct the parameters immediately from his computer into the training device, via the internet connection (the internet module for this program can be one of the many commercially available modules performing this task).
• In case of a connection via an Internet web site, the data recorded from the sessions performed by the patient are stored on a site especially dedicated for. A series of filters will help the manager of this control web center to track the sessions not performed properly or showing medical or technical problems. The manager can then contact, by either way possible, the doctor in charge to modify the program or to take any medical decision. In case nothing special is to be reported, which will be the vast majority of cases, the data of the performed sessions are immediately forwarded to the doctor in charge, directly in the patient's personal file at the doctor's office.
Both Internet connection AND memory card can work together. One modality does not exclude the other. This still gives the patient the opportunity to physically bring back his memory card to the doctor at the next medical visit even if the data will already be transferred via the Internet connection.

11. The customization of the training profile, using the system claimed previously, is unlimited. Today the 2 main used profiles are the steady state profile and the interval profile. One can easily build a crescendo-like or decrescendo-like or a random-like profile provided the input is the **HEART RATE**, and the output is the **WORKLOAD** in each phase of the session.

12. The memory card will also be able to carry special information related to special medical situation such as patient with arrhythmias or patient with hypertension. In those cases and ECG monitoring can be added to the control system. This an unlimited list
